Europäisches Patentamt

European Patent Office

Office européen des brevets

(19)

(11) Veröffentlichungsnummer: **0 012 443**
B1

(12) **EUROPÄISCHE PATENTSCHRIFT**

(45) Veröffentlichungstag der Patentschrift:
22.12.82

(21) Anmeldenummer: 79105189.9

(22) Anmeldetag: 14.12.79

(51) Int. Cl.³: **A 61 K 6/02**, A 61 K 35/32,
A 61 K 37/12, C 09 H 3/02

(54) Zahnpulpahöhlenfüllmaterial, bestehend aus Kollagen und Verfahren zu dessen Herstellung.

(30) Priorität: 16.12.78 DE 2854534

(43) Veröffentlichungstag der Anmeldung:
25.06.80 Patentblatt 80/13

(45) Bekanntmachung des Hinweises auf die Patenterteilung:
22.12.82 Patentblatt 82/51

(84) Benannte Vertragsstaaten:
AT BE CH DE FR GB IT LU NL SE

(56) Entgegenhaltungen:
DE-C 961 654
GB-A 1 068 587

CHEMICAL ABSTRACTS, Band 84, Nr 16, 19. April
1976, Seite 409, Spalte 2, NR. 111690d, Columbus,
Ohio, U.S.A.

(73) Patentinhaber: Intermedicat GmbH, Gerliswilstrasse 45,
CH-6020 Emmenbrücke (CH)

(72) Erfinder: Werner, Heinz-Helmut, Dr., Am Steig 2,
D-3508 Melsungen (DE)

(74) Vertreter: von Kreisler, Alek, Dipl.-Chem. et al,
Deichmannhaus am Hauptbahnhof, D-5000 Köln 1 (DE)

Zahnpulpahöhlenfüllmaterial, bestehend aus Kollagen und Verfahren zu dessen Herstellung.

Die Erfindung betrifft ein neues Zahnpulpahöhlenfüllmaterial, das aus Kollagen besteht.

In der Zahnheilkunde ist es sehr oft nötig, bei der Behandlung von erkrankten Zähnen die Zahnpulpahöhle zu öffnen. Um die geöffnete Zahnpulpahöhle wieder zu verschliessen, ist es bisher üblich und notwendig, eine Mehrschichtenfüllung einzubringen, die, von innen nach aussen aufgezählt, aus Calciumhydroxyd, aus Keramikmaterial und aus einer Deckfüllung besteht. Das seit etwa 1930 hierzu verwendete Calciumhydroxyd ermöglicht eine körpereigene Abkapselung der Zahnpulpahöhle gegen den Defekt. Das verwendete Calciumhydroxyd hat jedoch schwerwiegende Nachteile, da es eine alkalische Ätznekrose des vitalen Gewebes verursacht. Tiefer eingedrungene Partikeln von Calciumhydroxyd können so schwere Schäden verursachen, dass der Zahn nicht vital erhalten werden kann.

Ausserdem ist es schwierig, in die blutende Zahnpulpahöhle die richtige Schichtstärke von Calciumhydroxyd so einzubringen, dass nicht ein kleiner Bluterguss dort verbleibt, der leicht zu einer Infektion führen kann.

Die Erfindung stellt sich daher die Aufgabe, ein neues Material zu schaffen, das in beliebiger Menge verfügbar ist und in die Zahnpulpahöhle gefüllt werden kann, ohne Antikörper zu erzeugen und die Nachteile des Standes der Technik aufzuweisen.

Die Erfindung betrifft die in den Ansprüchen dargelegten Gegenstände.

Als Ausgangsmaterial für das erfindungsgemässe Zahnpulpahöhlenfüllmaterial dienen Knochen und insbesondere Bovines Knochenmaterial, z.B. vom Kalb. Es können jedoch auch andere Ausgangsmaterialien wie menschliche Knochen oder ähnliches kollagenhaltiges Material wie z.B. Sehnen und Häute verwendet werden.

Nachstehend wird das Verfahren zur Herstellung des erfindungsgemässen Zahnpulpahöhlenfüllmaterials anhand von Kalbsknochen als Ausgangsmaterial beispielhaft dargestellt. Das Verfahren stellt eine Kombination von verschiedenen Stufen dar, die in der angegebenen Reihenfolge durchgeführt werden müssen, um das erfindungsgemässe Zahnpulpahöhlenfüllmaterial zu erhalten.

In einer ersten Stufe werden die Knochen zur Entfernung des Blutfarbstoffes und anderer wasserlöslicher Eiweisse über einen gewissen Zeitraum gewässert. Vorzugsweise beträgt dieser Zeitraum 2–3 Tage. Bei diesem Wässern wird die Waschflüssigkeit häufig erneuert. Das Wässern kann auch als fliessendes Wässern durchgeführt werden. Eine besondere Temperatur ist nicht erforderlich, doch wird zweckmässig bei Umgebungstemperatur gewässert.

Eine Zweite Verfahrensstufe dient der Entfernung weiterer, nicht wasserlöslicher Eiweissanteile. Es ist wichtig, dass fremde Eiweisse bzw. Proteine ausserhalb des Kollagens wie z.B. Knochenzellen entfernt werden, um Antireaktionen durch Antikörperbildung auch enzymatischer Art zu vermeiden. Die Entfernung der Eiweissanteile in dieser Stufe erfolgt durch Einlegen des Knochenmaterials in eine $H_2O_2$-Lösung. Diese $H_2O_2$-Lösung sollte eine Konzentration von über 10% aufweisen. Die Zeitdauer der Behandlung beträgt normalerweise 2–3 Tage. Auch hier muss die Lösung mehrfach erneuert werden, um eine vollständige Entfernung der Eiweissanteile zu gewährleisten.

Die dritte Verfahrensstufe besteht wieder in einem Spülschritt, bei dem das in der zweiten Stufe erhaltene Material mit kaltem Wasser zur Auswaschung und Abführung der gelösten Weichteile gewässert wird. Hier ist das fliessende Wässern von Vorteil.

In der vierten Verfahrensstufe wird das so erhaltene Material entfettet. Das Entfetten erfolgt mit Hilfe von organischen Lösungsmitteln. Geeignete Lösungsmittel sind z.B. Aceton oder Äther oder insbesondere eine Kombination beider Lösungsmittel. Die Entfettung kann in einer beliebigen dafür geeigneten Apparatur vorgenommen werden. Besonders geeignet ist eine Soxhlet-Apparatur für diese Entfettung.

In der fünften Stufe des Verfahrens wird das in der vierten Stufe erhaltene Material mit Hilfe von Komplexbildnern oder Ionenaustauschern behandelt. Diese Stufe dient dem Zweck, den Mineralanteil des Knochens durch Komplexbildung herauszulösen. Geeignete Komplexbildner sind z.B. die Salze von Äthylendiamintetraessigsäure.

Eine geeignete Lösung kann nach einem in Romeis «Lehrbuch der mikroskopischen Technik» angegebenen Verfahren hergestellt werden Dabei werden 250 g Dinatriumäthylendiamintetraessigsäure mit 200 ml destilliertem Wasser versetzt und erwärmt. Unter dauerndem Rühren werden 50 ml einer 40%igen Natronlauge zugesetzt und das Ganze mit destilliertem Wasser auf 800 ml aufgefüllt. Durch tropfenweisen Zusatz von 40%iger Natronlauge wird auf pH 7,4 eingestellt und mit destilliertem Wasser auf 1000 ml Gesamtvolumen aufgefüllt.

Das Knochenmaterial wird in eine solche Lösung eingelegt und verbleibt darin eine geraume Zeit, z.B. bis zu mehreren Wochen, bis ein kautschukartiges elastisches Produkt entstanden ist.

Das so erhaltene kautschukartige elastische Produkt wird in einer sechsten Verfahrensstufe erneut gewaschen, um die vorhandenen Salzreste zu entfernen. Das Waschen geschieht durch fliessendes Wässern für einen ausreichenden Zeitraum, wie z.B. 2–3 Tage.

Das dabei erhaltene Material wird dann in einer siebten Verfahrensstufe gegebenenfalls einer Gefriertrocknung in üblicher Weise unterworfen.

Das erhaltene Material wird dann in einer achten Verfahrensstufe der üblichen Sterilisation insbesondere der Strahlensterilisation unterworfen.

Die Strahlensterilisation kann mit üblichen Dosen z.B. mit einer Dosis von 2,5 Megarad erfolgen.

Durch das erfinderische Verfahren ist es möglich geworden, ein Zahnpulpahöhlenfüllmaterial aus gereinigtem Kollagen und insbesondere gereinigtem Kalbskollagen zu erhalten, das durch sorgfältige Vermeidung aller schädigenden Faktoren wie z.B. Säure beim Entkalkungsprozess, in seiner molekularen Struktur weitgehend erhalten ist.

Das so erhaltene Grundmaterial zeigt beim Einbringen in die Zahnpulpahöhle als Füllmaterial erstaunlich gute Eigenschaften. Beispielsweise ist die Resorptionszeit wesentlich höher als bei anderen bisher bekannten Materialien.

Darüber hinaus haben Untersuchungen, die bei Versurchstieren zu verschiedenen Zeitpunkten durchgeführt wurden, eine hervorragende Verträglichkeit ohne Abstossungsreaktionen des Körpers gezeigt. Feingewebliche Untersuchungen zeigten Einlagerungen von Mineralsubstanz, Bindegewebszellen, einsprossende Blutgefässe und pulpabildende Zellen.

Mit dem erfindungsgemässen Zahnpulpahöhlenfüllmaterial aus nach obigem Verfahren hergestellten Kalbskollagen stellte sich heraus, dass sich die Nachteile des Standes der Technik alle vermeiden lassen. Die am Zahn notwendige Abkapselung der Pulpahöhle entsteht nach Einbringen von Kollagen durch Verknöcherung des implantierten Füllmaterials. Die biologische Verträglichkeit ist wesentlich besser. Es kommt nicht zur Ausbildung von Nekrosen, d.h. der Zahn ist weniger geschädigt als beim bisherigen Verfahren.

Feingewebliche Untersuchungen mit dem Lichtmikroskop, elektronenoptische Untersuchungen und Fluoreszenzuntersuchungen mit Vitalfarbstoffen an Schliffpräparaten solcher Zähne erbrachten den Nachweis der hervorragenden Verträglichkeit von Kalbskollagen mit fortschreitender Verknöcherung nach dem Einbau und damit besserer Behandlungsergebnisse als nach dem bisherigen Verfahren möglich war. Besonders günstig ist hierbei auch die bekannte blutstillende Wirkung von nativem Kollagen, die die Ausbildung von Blutergüssen im Zahn verhindert und damit die Komplikationsrate dieser Behandlungsmethode weiter senkt.

Das auf obige Weise erhaltene Grundmaterial kann in seinen hervorragenden Eigenschaften als Zahnpulpahöhlenfüllmaterial noch wesentlich verbessert werden, wenn man dieses Material mit einer geringen Menge des Hormons Calcitonin, auch Thyreo-Calcitonin genannt, vermischt. Beispielsweise eignen sich dazu im Handel erhältliche Calcitonin-Lösungen. Die wesentlich verbesserten Ergebnisse lassen den Schluss zu, dass es sich hier um einen synergistischen Effekt zwischen dem Zahnpulpahöhlenfüllgrundmaterial und dem Hormon Calcitonin handelt. Die Substanzneubildungszeit liess sich mit einem derart mit Calcitonin versetzten Füllmaterial weiter erheblich verkürzen.

Die Erfindung wird nachstehend anhand eines Ausführungsbeispiels erläutert:

1 kg Kalbsknochen (Spongiosastücke) wird sorgfältig gewaschen und so von groben Blutresten befreit. Diese Knochenmenge wird dann in einem geeigneten Gefäss mit Zu- und Abflussmöglichkeit gelagert und für 72 Stunden durch fliessendes Leitungswasser einer Dauerspülung ausgesetzt. Dann wird es in eine $H_2O_2$-Lösung (15%) für 72 Stunden eingelegt. Die Lösung wird 3- bis 4mal gewechselt. Die Behandlung mit fliessendem Leitungswasser wird dann für 24 Stunden wiederholt. Anschliessend wird die Entfettung in einer Soxhlet-Apparatur mit 60% Aceton und 40% Diäthyläther über ca. 12 Stunden vorgenommen.

1250 g Dinatriumäthylendiamintetraessigsäure werden mit 1000 ml destilliertem $H_2O$ versetzt und erwärmt. Unter dauerndem Rühren werden 200 ml NaOH (40%) zugesetzt und das Ganze mit destilliertem Wasser auf 4000 ml aufgefüllt. Durch tropfenweisen Zusatz von 40%iger NaOH wird auf pH 7,4 eingestellt und mit destilliertem Wasser auf 5000 ml aufgefüllt.

In die so erhaltene Lösung werden die wie vorstehend beschrieben behandelten Knochen eingelegt, bis mehrere entnommene Proben sich als mineralfrei erweisen. Bei Zimmertemperatur sind dazu je nach Grösse der Einzelstücke etwa 3–4 Wochen erforderlich.

Anschliessend wird gefriergetrocknet. Danach wird nach geeigneter Folienverpackung durch Bestrahlen mit 2,5 Mrad sterilisiert.

Im folgenden werden Versuche und ihre Ergebnisse beschrieben, bei denen das Knochenersatzmaterial gemäss der Erfindung implantiert worden ist.

Bei der Einpflanzung in die Pulpahöhle des Zahnes wurde die Entwicklung von Knochengewebe nach der Einpflanzung der nach obigem Verfahren hergestellten Kollagenstücke nachgewiesen.

Bei einer experimentellen Prüfung an Beaglehunden konnte gezeigt werden, dass durch das konventionelle Verfahren die Zahnkonservierung unter Erhaltung der vitalen Pulpa nicht nur ersetzt werden konnte, sondern dass das Ausmass der durch den Eingriff bedingten Schädigung des Zahnes wesentlich verringert wurde.

**Patentansprüche**

1. Zahnpulpahöhlenfüllmaterial, dadurch gekennzeichnet, dass es aus natürlichem Kollagen besteht, das in seiner molekularen Struktur weitgehend erhalten ist und durch das folgende Verfahren hergestellt worden ist:
a) Entfernung des Blutfarbstoffes und anderer wasserlöslicher Eiweisse aus natürlichem kollagenhaltigem Material wie Knochen durch Behandeln mit Wasser;
b) Entfernung von Eiweiss und Proteinen durch mehrmaliges Behandeln mit $H_2O_2$-Lösung einer Konzentration von mindestens 10%;
c) Spülen in kaltem Wasser;
d) Entfettung mit organischen Lösungsmitteln;
e) Herauslösung der Mineralanteile des Kollagenmaterials durch Komplexbildner oder Ionenaustauscher;

f) Restsalzentfernung durch fliessendes Wässern;

g) gegebenenfalls Gefriertrocknen in üblicher Weise und

h) Sterilisation in üblicher Weise, insbesondere Strahlensterilisation,

und dass es gegebenenfalls das Hormon Calcitonin enthält.

2. Zahnpulpahöhlenfüllmaterial nach Anspruch 1, dadurch gekennzeichnet, dass als Ausgangsmaterial Bovines-Knochenmaterial, z.B. Knochen vom Kalb, verwendet werden.

3. Zahnpulpahöhlenfüllmaterial nach Anspruch 1 und 2, dadurch gekennzeichnet, dass es zusätzlich das Hormon Calcitonin enthält.

4. Verfahren zur Herstellung von Zahnpulpahöhlenfüllmaterial nach Ansprüchen 1 und 2, dadurch gekennzeichnet, dass man

a) den Blutfarbstoff und andere wasserlösliche Eiweisse aus dem natürlichen kollagenhaltigen Material wie Knochen durch Behandeln mit Wasser entfernt;

b) Eiweiss und Proteine durch mehrmaliges Behandeln mit einer $H_2O_2$-Lösung mit einer Konzentration von mindestens 10% entfernt;

c) mit kaltem Wasser spült;

d) mit organischen Lösungsmitteln entfettet;

e) die Mineralanteile des Kollagenmaterials durch Komplexbildner oder Ionenaustauscher herauslöst;

f) das erhaltene Material zur Entfernung des Restsalzes in fliessendem Wasser wässert;

g) das erhaltene Material gegebenenfalls in üblicher Weise gefriertrocknet und

h) das erhaltene Material in üblicher Weise sterilisiert, insbesondere durch Strahlensterilisation.

## Claims

1. A tooth pulp cavity filling material, characterized in that it consists of natural collagen, which is preserved to a large extent as to its molecular structure and has been produced by the following process:

a) removing the blood pigment and other water-soluble proteins from natural collagen-containing material such as bones by treatment with water;

b) removing proteins by several treatments with a $H_2O_2$ solution having a concentration of at least 10%;

c) rinsing in cold water;

d) degreasing with organic solvents;

e) evaluating the mineral portions of the collagen material by complexing agents or ion exchangers;

f) removing the residual salts by rinsing in flowing water;

g) optionally freeze-drying in the usual manner; and

h) sterilizing in the usual manner, particularly by radiation sterilization,

and that it optionally contains the hormone calcitonin.

2. A tooth pulp cavity filling material according to claim 1, characterized in that a bovine bone material such as calf bones is used as the starting material.

3. A tooth pulp cavity filling material according to claim 1, characterized in that it additionally contains the hormone calcitonin.

4. A process for producing a tooth pulp cavity filling material according to claims 1 and 2, characterized in that

a) the blood pigment and other water-soluble proteins are removed from the natural collagen-containing material such as bones by treatment with water;

b) proteins are removed by several treatments with a $H_2O_2$ solution having a concentration of at least 10%;

c) there is rinsed in cold water;

d) there is degreased with organic solvents;

e) the mineral portions of the collagen material are evaluated by complexing agents or ion exchangers;

f) the obtained material is treated in flowing water in order to remove the residual salts;

g) the obtained material is optionally freeze-dried in the usual manner; and

h) the obtained material is sterilized in the usual manner, particularly by radiation sterilization.

## Revendications

1. Matière d'obturation des cavités dans la pulpe dentaire, caractérisée en ce qu'elle consiste en un collagène naturel qui est obtenu principalement dans sa structure moléculaire et a été préparé par le procédé qui consiste à:

a) séparer l'hémoglobine du sang et les autres protéines hydrosolubles d'une matière naturelle contenant du collagène, telle que des os, par traitement avec de l'eau;

b) séparer l'albumine et les protéines par traitement répété avec une solution de $H_2O_2$ à une concentration d'au moins 10%;

c) rincer à l'eau froide;

d) dégraisser avec des solvants organiques;

e) séparer les parties minérales de la matière collagènique par un agent complexant ou un échangeur d'ion;

f) séparer le sel résiduel par l'eau courante;

g) éventuellement lyophiliser de façon usuelle, et

h) stériliser de façon usuelle, en particulier par irradiation,

et qu'elle contient éventuellement l'hormone dite calcitonine.

2. Matière d'obturation des cavités dans la pulpe dentaire selon la revendication 1, caractérisée en ce qu'on utilise comme matière première une matière osseuse de bovins, par exemple des os de veaux.

3. Matière d'obturation des cavités dans la pulpe dentaire selon les revendications 1 et 2, caractérisée en ce qu'elle contient en outre l'hormone dite calcitonine.

4. Procédé pour la préparation d'une matière d'obturation des cavités dans la pulpe dentaire selon l'une quelconque des revendications 1 et 2, caractérisé en ce qu'il consiste à:

a) séparer l'hémoglobine du sang et les autres protéines solubles dans l'eau de la matière naturelle contenant du collagène tel que des os, par traitement avec de l'eau;

b) séparer l'albumine et les protéines par traitement répété avec une solution de $H_2O_2$ à une concentration d'au moins 10%;

c) rincer à de l'eau froide;

d) dégraisser avec des solvants organiques;

e) éliminer les parties minérales de la matiére collagènique par un agent complexant ou un échangeur d'ion;

f) laver la matière obtenue à l'eau courante pour séparer le sel résiduel;

g) éventuellement lyophiliser de façon usuelle la matière obtenue, et

h) stériliser de façon usuelle la matière obtenue, en particulier par irradiation.